# EUROPEAN PATENT APPLICATION

(11) **EP 2 193 744 A1**
(43) Date of publication of application: **09.06.2010**
(21) Application number: 09175820.1
(22) Date of filing: 12.11.2009
(51) Int. Cl.: A61B 5/00, A61B 5/02, G06F 19/00

(54) **System and method for dynamically configuring functionality of remote health monitoring device**

(30) Priority: 17.11.2008 US 272002
(71) Applicant: Honeywell International Inc., Morristown, NJ 07962 (US)
(72) Inventor: Vayalattu, Ginto Kurian, 560078, Bangalore, Karnataka (IN); Nair, Manoj Thankappan, 560093, Bangalore, Karnataka (IN); Thatha, Sudeesh, 560076, Bangalore, Karnataka (IN)
(74) Representative: Buckley, Guy Julian

(57) **Abstract**

A system for monitoring vital signs of an individual functions in accordance with a predetermined monitoring profile. The profile can be automatically revised by a management system in accordance with vital signs data, monitor operational information and manual inputs from a care provider to form a revised profile. The revised profile can be can be approved and then transferred to the monitoring system.

## Description

### FIELD

The invention pertains to patient or resident monitoring units, or, systems. More particularly, the invention pertains to such systems where revised or updated operating characteristics of the respective monitoring system(s) can be remotely established and then downloaded to the respective monitoring unit.

### BACKGROUND

Various types of monitoring systems of physiological conditions of an individual are known. Some of these monitoring systems are intended to be used by individuals in their residences on an on-going daily basis to implement home based disease management programs.

Such systems operate in accordance with one or more pre-established programs in carrying out their monitoring functions. Data, for example vital signs, from the monitoring process can be transmitted to a remote location for storage and review by care givers.

The care givers can determine wellness, or medical condition, from a review of an individual's stored data. Feedback can be provided to the individual as to changes in medications and/or other behaviors to try to maximize the individual's health, or carry out long-term disease management for example.

Representative monitoring systems are disclosed in "In-Residence Monitoring System Incorporating Voice Output" U.S. Ser. No. 11/226,550 filed September 14, 2005; "Monitoring System for a Residence" U.S. Ser. No. 11/189,332 filed July 26, 2005; and "Residential Monitoring System for Selected Parameters" U.S. Ser. No. 11/119,182 filed April 29, 2005 all of which are assigned to the Assignee hereof and incorporated herein by reference.

There is a continuing need to be able to revise or adjust the monitoring programs as an individual's condition evolves over time. It would be desirable to be able to automatically establish a revised program based on past vital signs, and/or other behavioral information which have been collected over a period of time for an individual.

### BRIEF DESCRIPTION OF THE DRAWINGS

Fig. 1 is a block diagram that illustrates aspects of the invention; and

Fig. 2 is another block diagram that discloses additional aspects of the invention.

### DETAILED DESCRIPTION

While embodiments of this invention can take many different forms, specific embodiments thereof are shown in the drawings and will be described herein in detail with the understanding that the present disclosure is to be considered as an exemplification of the principles of the invention, as well as the best mode of practicing same, and is not intended to limit the invention to the specific embodiment illustrated.

For effective disease management and monitoring of chronically ill patients (or) aged patients, it is desirable to monitor various vital parameters in combination with each other. Even though there are agencies that are continuously monitoring the Patient's vital data, it is a very time consuming operation to dynamically change the functionality (or) behavior of the monitor unit by considering the Patient's previous data. In accordance herewith, exemplary functionality changes are achieved by either inclusion or exclusion of a medical sensor during Vital Acquisition sequence. Exemplary changes to the parameters or the behavior of the monitoring unit correspond to modification of the Vital Acquisition sequence (or) Communication mechanism, etc.

Embodiments of the invention include a programmable monitoring unit which would be located at the home or residence of an individual whose health parameters were to be monitored. A displaced data storage unit can be coupled to the monitoring unit via wireless or wired communications links such as the switched telephone network or the Internet.

A configuration system is coupled to both the monitoring unit and the storage unit. The configuration system can, responsive to physiological information, such as vital signs, functional information, such as the existing program, or, parameters for the monitoring unit, or input from health care professionals, automatically generate a proposed updated program, operating parameters, or operating profile for the monitoring unit. The proposed operating profile can be reviewed for acceptability by one or more health care professionals. Accepted profiles can be transmitted to the respective monitoring unit and automatically installed therein to provide updated functionality.

Aspects of a method in accordance herewith include, automatically generating the proposed, or, recommended monitor configuration(s). Transmitting approved monitor configuration(s) to the respective monitor unit, and, installing the suggested configuration in the monitor unit without user intervention. The monitoring process is then implemented with the updated configuration.

As discussed above, after the vital signs acquisition sequence the monitor forwards the vital signs data and available diagnostic information (any errors, time taken for each measurement, communication issues, if any, etc.) to a system server for storage. Having this data available at one or more centralized servers, makes it readily available to health care professionals who are keeping track of the individual's condition as well as control and analysis circuitry.

The control and analysis circuitry can analyze the data by considering previous medical history, diagnostic information and also any monitor unit configuration profiles created by the health care professional. After its analysis, it automatically generates a recommended monitor configuration which will be presented for clinical staff approval.

Various components involved in the configuration generation process are discussed below along with information as to the functionality of each.

Data storage and professional input/output system: This system collects all the data from the monitor unit including vital signs collected and monitor diagnostic information. The interface enables health care professionals to access this data, and to propose configuration changes to the monitoring unit as appropriate.

Configuration generation system: This system processes the inputs from both the Health Care Professional as well as the data storage system. This system will consider the monitor function or configuration related inputs from the Health Care Professional if there is a change else the monitor function or configuration related information from the storage and interface system is considered. The vital signs information would be compared to any configuration information proposed by the Health Care professional.

Configuration Profiles: This process could collect data from configured profiles or inputs from the Health Care professional for both Vitals to be recorded as well as the monitor unit's functionality or program. Configurations created or proposed previously by the Health Care professional could be used. Alternately, the monitor unit's operational plan or program could be configured dynamically at that instant based on an analysis of data collected from the monitor unit or system.

Exemplary configuration revisions could include, without limitation:
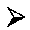 Send the configuration to the monitor unit for retest, if any of the vital parameter values are lower than suggested clinical reference values.
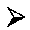 Ignore the ECG vital acquisition, if the BP & Glucose limits are with in the limits.
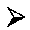 Enable the Peak Flow sensor, if the health care professional identifies the need of this vital.

Hence, in embodiments of the invention, either the configuration generation system, or, Clinical Staff can establish the recommended configuration considering the patient's medical history. As explained above, periodically the configuration generation system establishes a proposed configuration pattern to be used by the monitor unit, or system, considering the individual's previous medical history. Once the system automatically generates the recommended pattern for the monitor, it sends a notification to the associated Clinical Staff. Clinical staff then can review the previous configuration and the recommended configuration along with the history data and accept or further revise the proposed plan.

Following is an example of structure of a Configuration Packet which could be transmitted to a monitoring unit:

```
          <Config_Packet>
           <Sensors>
            <SCALF>1</SCALF>
            <OXIMETER>1</OXIMETER>
            <TEMPERATURE>1</TEMPERATURE>
            <QUESTIONS>1</QUESTIONS>
            <ECG>1</ECG>
            <GLUCOSE_METER>K/GLUCOSE_METER>
            <PT_MONITOR>1</PT_MONITOR>
            <PEAK_FLOW>1</PEAK_FLOW>
            <NIBP>1</NIBP>
           </Sensors>
           <Behaviour>
            <POTS_GPRS>1</POTS_GPRS>
            <SVALIDATION>1</SVALIDATION>
            <REPORTING_TIME>1</REPORTING_TIME>
              <SETUP_WIZARD>1</SETUP_WIZARD>
            <EDU_CONTENT>1</EDU_CONTENT>
            <P_REMINDERS>1</P_REMINDERS>
            <PARTIAL_RETEST>1</PARTIAL_RETEST>
            <REMOTE_FIMWARE_DWLD>1</REMOTE_FIRMWARE_DWLD>
            <ANIMATION>1</ANIMATION>
           </Behaviour>
          </Config_Packet>
```

Once clinical staff provides their acceptance, the system forwards the approved configuration for transmission to the Monitor. For transmission, a variety of communication channels can be used, without limitation. These include POTS, wireless, the Internet, or physical memory updates, etc.

Once monitor receives the recommended configuration information, it installs the configuration. Once the monitor installs the configuration, it validates the syntax & semantics of the packet. After this process, it checks for any new functionality or behavior and if present, it configures itself automatically. Then it checks and updates Sensor/Vital configuration information. Having these updated configuration settings; it follows the suggested configurations during Vital Acquisition sequence. This process takes into account changing health parameters of the individual.

The proposed processes of monitor configuration are based on the Patient Disease profile and previous history. In summary:
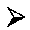 Dynamically changing the Health Monitor configurations
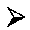 Usage of Health Monitor Diagnostic for capturing Monitor usability
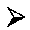 Use of diagnostic information by the configuration generation system in providing a more effective monitoring process for the individual or patient
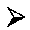 Dynamic generation of a recommended configuration
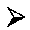 Role based workflow for applying the recommended configuration to a specified category (or) group of monitors

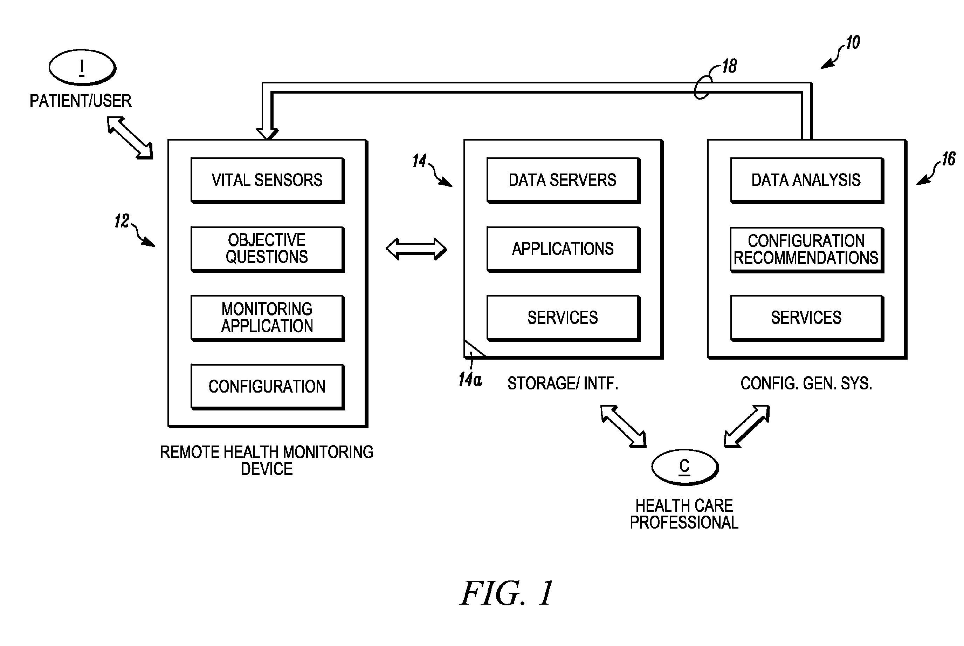

Fig. 1 is a block diagram 10 of a system in accordance with the invention. Physiological conditions, such as blood pressure, temperature, glucose levels, blood oxygen levels or the like, without limitation, are monitored by a health monitoring device or system 12 (examples of which were noted above). System 12 is in at least intermittent communication with a storage/health care professional interface unit 14.

Unit 14 is coupled to configuration generation system 16. The respective health care professional C can communicate with system 14 to review and evaluate patient history, such as stored vital signs and other information related to the individual I along with operating information as to the monitoring unit 12 via a graphical user interface 14a.

System 16 in response to stored data concerning the individual I along with input from the professional C can automatically generate some, or all of a proposed monitor operational profile for the device 12. That proposed profile can be reviewed and approved or modified by professional C and then transmitted, via link 18 to the device 12. This updated profile can then be the basis of further monitoring of parameters indicative of the health of the individual I.

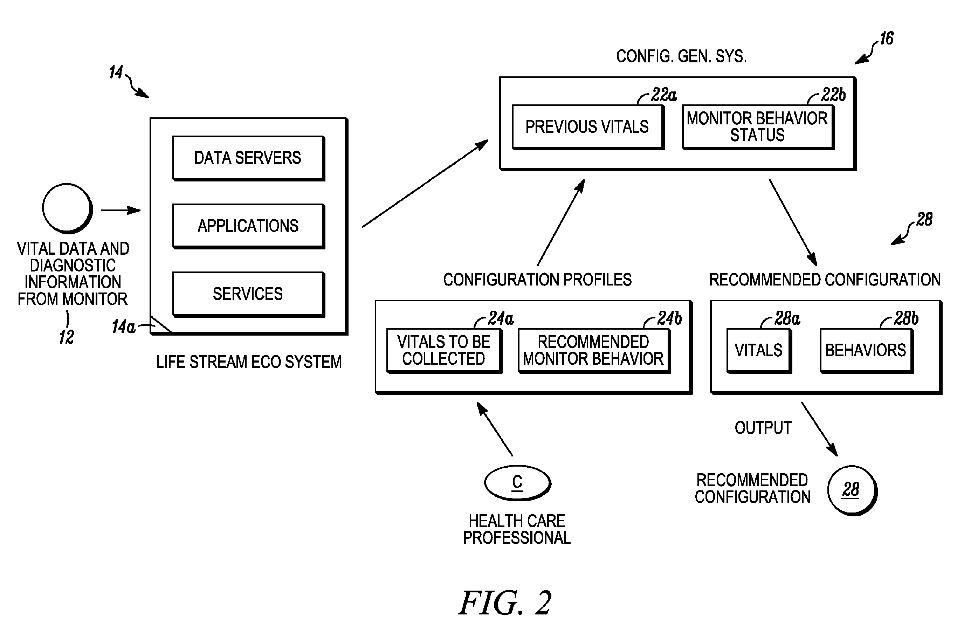

Fig. 2 illustrates further details of the system and method of Fig. 1. As illustrated in Fig. 2 the configuration generation system 16 takes into account previously obtained vital signs data as well as monitor parameter settings, 22a,b and inputs 24a,b from professional C to automatically generate a recommended configuration 28 specifying vital signs information 28a, and monitor parameter characteristics 28b.

Those of skill will understand that the units 12, 14, 16 can all be implemented with one or more programmable processors and associated control software which when executed by the respective processor, or processors, provide the above described functionality. None of the specific details of such implementations are limitations of the invention.

From the foregoing, it will be observed that numerous variations and modifications may be effected without departing from the spirit and scope of the invention. It is to be understood that no limitation with respect to the specific apparatus illustrated herein is intended or should be inferred. It is, of course, intended to cover by the appended claims all such modifications as fall within the scope of the claims.

## Claims

1. A health monitoring apparatus comprising:
a monitoring device which includes a plurality of sensors of physiological conditions of an individual, the device automatically monitoring the individual in accordance with a predetermined monitoring profile, the device has an input/output port for receiving at least a portion of the predetermined profile from a displaced source, and for sending information from the sensors to a displaced storage system where the storage system is coupled to at least one visual, graphical, input/output interface for manual review and evaluation of the information received from the monitoring device; and
analysis and configuration circuits coupled to the storage system and to the input/output interface with the analysis and configuration circuits, responsive to stored information from the monitoring device and inputs from the input/output interface, to automatically establish a revised monitoring profile.

2. An apparatus as in claim 1 where the analysis and configuration circuits include output circuits to transmit the revised monitoring plan to the monitoring device.

3. An apparatus as in claim 2 where the monitoring device includes circuits to substitute at least part of the revised profile for the predetermined profile at the monitoring device.

4. An apparatus as in claim 1 where the sensors for the monitoring device are selected from a class which includes at least temperature sensors, cardiac sensors, blood pressure sensors, respiratory sensors, oxygen sensors and glucose sensors.

5. An apparatus as in claim 1 where the analysis and configuration circuits respond to information received from the monitoring device, diagnostic information and at least one prior configuration profile to automatically generate the revised monitoring plan.

6. An apparatus as in claim 5 where the analysis and configuration circuits include circuitry that forwards the revised monitoring profile to an input/output interface for review.

7. An apparatus as in claim 6 where the analysis and configuration circuits include output circuits to transmit the revised and reviewed monitoring profile to the monitoring device.

8. An apparatus as in claim 7 where the monitoring device implements the revised and reviewed monitoring profile upon receipt.

9. A monitoring apparatus comprising:
a monitoring device which includes a plurality of sensors of physiological conditions of an individual, the device automatically monitoring the individual in accordance with a predetermined monitoring profile, the device has an input/output port for receiving at least a portion of the predetermined profile from a displaced source, and for sending information from the sensors to a displaced storage system where the storage system is coupled to at least one visual, graphical, input/output interface for manual review and evaluation of the information received from the monitoring device; and
a profile proposing system which is coupled to the storage system and a graphical user interface, the profile proposing system receives profile related inputs from health care providers via the interface, as well as from patient related data from the monitoring device and generates, in response thereto, a recommended configuration including specifying sensor related information and monitoring device functionality where the recommended configuration is presented for review at the interface.

10. A method comprising:
establishing a plan for monitoring at least a plurality of physiological conditions of an individual;
monitoring the individual for a period of time in accordance with the plan;
sending information as to the monitored conditions of the individuals to a displaced location; and
processing the information and selected additional inputs, associated with the individual, to automatically establish a revised monitoring plan.

11. A method as in claim 10 which includes evaluating the revised plan, and manually modifying the revised plan to form an approved plan.

12. A method as in claim 11 which includes transferring the revised plan to a predetermined location and thereafter, monitoring the individual for a second period of time in accordance with the revised plan.

13. A method as in claim 11 where manually modifying includes modifying the revised plan in accordance with physiological conditions of the individual.

14. A method as in claim 10 which includes obtaining recommended inputs for the revised plan prior to automatically establishing the revised monitoring plan.

15. A method as in claim 14 which includes manually evaluating the revised plan, and, responsive thereto, establishing an approved plan.

16. A method as in claim 15 which includes transferring the approved plan to a predetermined location and thereafter, monitoring the individual for a second period of time in accordance with the approved plan.
